# EUROPEAN PATENT SPECIFICATION

(11) **EP 3 236 909 B1**
(45) Date of publication and mention of the grant of the patent: **11.12.2024**
(21) Application number: 15787270.6
(22) Date of filing: 23.09.2015
(51) Int. Cl.: A61F 13/533, A61F 13/15

(54) **PARTICLE ENTRAINED AIR-PERMEABLE STRUCTURES**
LUFTDURCHLÄSSIGE STRUKTUREN MIT MITGEFÜHRTEN PARTIKELN
STRUCTURES PERMÉABLES À L'AIR CHARGÉES DE PARTICULES

(30) Priority: 28.12.2014 GB 201423274
(43) Date of publication of application: 01.11.2017
(73) Proprietor: Optimum Technology Intellectual Property LLC, Douglas Isle of Man IM1 1JA (GB)
(72) Inventor: PATCHETT, Kim, Essex RM17 5RH (GB)
(74) Representative: Mewburn Ellis LLP
(86) International application number: PCT/GB2015/052746
(87) International publication number: WO 2016/108039

(56) References cited:
- EP-A1- 1 870 067
- EP-A1- 1 920 744
- WO-A1-2013/152809
- GB-A- 2 504 783

## Description

This disclosure relates to a method for dissipating and entrapping absorbent particles within air-permeable, non-woven structures. In particular, it relates to such a method for dissipating and entrapping particles that in their manufactured or synthesized state have a mean particle diameter of between about 10µm and 1000µm. It will be noted however that the disclosed method may be similarly practiced with particles having a mean diameter smaller than 10µm and larger than 1000µm.

Absorbent particles suitable for processing according to the disclosed method include but, are not limited to: sodium polyacrylate, polyacrylamide copolymer, ethylene maleic anhydride copolymer, cross-linked carboxymethylcellulose, polyvinyl alcohol copolymers, cross-linked polyethylene oxide, and starch grafted copolymers of polyacrylonitrile, either solely, or in combination.

In recent years, there has been a trend in the hygiene industry to reduce the mass of fluff pulp contained within hygiene products such as infant diapers, adult incontinence products and feminine hygiene products. The trend to reduce the mass of fluff pulp within hygiene products is directed at using less mass of raw materials in the end product thereby both reducing the manufacturing demand for raw materials, and reducing the amount of soiled materials for disposal after the product has been used for its intended purpose. A further trend to reduce the mass of fluff pulp with hygiene products is directed at reducing or eliminating the atmospheric dust within end product manufacturing plants emanating from the processing of the fluff pulp.

The conventional structure of, for example, an infant diaper has at its core a combined mass of fluff pulp and absorbent particles known as SAP (super absorbent polymers). Conventional methods for combining fluff pulp and SAP result in the SAP being randomly distributed throughout the mass of the fluff pulp core. Generally, the SAP comprises sodium polyacrylate, which may be acquired from a number of commercial sources. Although each commercially available grade of sodium polyacrylate will have a different particle size distribution (PSD) when compared to another grade or a different manufacturer, a typical PSD is shown below:

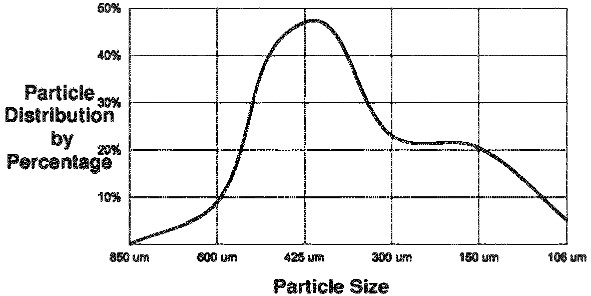

Airborne particles have irregular shapes, and their aerodynamic behaviour is expressed in terms of the diameter of an idealised spherical particle known as the aerodynamic diameter. Particles are sampled and described on the basis of their aerodynamic diameter, which is usually simply referred to as particle size. Particles having the same aerodynamic diameter may have different dimensions and shapes. The aerodynamic diameter of an irregular particle is defined as the diameter of the spherical particle with a density of 1000 kg m⁻³ and the same settling velocity as the irregular particle.

It is well understood that particles having an aerodynamic diameter of between 0.5µm and 10µm may easily settle within the transfer region of the human lungs (the alveoli) and may lead to acute or chronic ill health effects depending upon the type of powder and its specific chemical and physical composition. Particles of aerodynamic diameter in the range of 0.5µm and 10µm are defined as being within the respirable range. Particles of a size below the respirable range may be exhaled naturally after entering the lungs. Particles of a size above the respirable range are removed by the impingement of nasal hairs and very fine hairs (cilia) that line the bronchi and trachea and trap foreign bodies within the respiratory system. Trapped foreign bodies are covered in mucus and passed out up into the throat where they are swallowed, sneezed or spat out. Mucus ensures that particles do not become re-entrained in the inhalation flow and ultimately particles are discharged from the body thereby protecting the lungs from particle ingress.

In the United Kingdom, the threshold limit value (TLV) applied to particles within the respirable range have been published by the Health and Safety Executive (HSE). The Control of Substances Hazardous to Health (COSHH) Act 1988, introduced the Maximum Exposure Limit (MEL) and the Occupational Exposure Standard (OES).

Historic TLV values and the level of hazard that they pose are as follows:
Group 1 - very dangerous, 0µg m⁻³ to 50µg m⁻³. Readily give rise to fibrosis and includes: beryllium, silica in the cristobalite form and blue asbestos (5 fibres/cm³ and less than 5µm in length). Note that asbestos is not classified using aerodynamic diameter due to its unusual fibre like shape;
Group 2 - dangerous, 50µg m⁻³ to 250µg m⁻³. Includes: asbestos (other forms of and with 5 fibres/cm³ > 5µm in length), silica such as quartz and mixed powders containing 20% or more of silica;
Group 3 - moderate risk, 250µg m⁻³ to 1000µg m⁻³. Includes: mixed powders of < 20% silica, talc, mica, kaolin, cotton, organic dust, graphite and coal; and
Group 4 - low risk, >1mg m⁻³, includes: cement powder, limestone, glass, barites, perlite, iron oxide, magnesia and zinc oxide.

Of course, sensible precautions such as the wearing of suitable dust masks within the working environment may protect against the ingress of particles within the respirable range into the lungs but in addition to the human health hazards associated with processing small particles, it may also be the case that contamination of the end product in manufacture or the production machinery itself may be problematic.

Consequently, it is desirable to create a process for eliminating fluff pulp and other potential dust hazards, and in particular those particles that fall within the respirable range of between 0.5µm and 10µm.

Coincidentally, in the case of infant diapers, adult incontinence products and feminine hygiene products, a reduction in the mass of fluff pulp within the product also increases the number of finished and packaged products that can be transported within a given volume and therefore reduces transport costs per product unit shipped. This therefore has a beneficial impact on both reducing air pollution and reducing carbon dioxide emissions from burning fossil fuels during transport.

It would therefore be beneficial if fluff pulp could be completely eliminated from within the core structure of infant diapers, adult incontinence products and feminine hygiene products. It would be further beneficial if the SAP were distributed within a fluff pulp free, air-permeable core in a controlled manner, such that the different sizes of particles within a specific grade of SAP were positioned to absorb liquid waste in the most efficient manner rather than, as is currently the case, where the different sizes of SAP particles within a specific grade are randomly distributed throughout the core.

The present invention therefore seeks to provide a method for managing absorbent particles within a fluff pulp free, air-permeable, non-woven absorbent structure, for use within infant diaper, adult incontinence products, feminine hygiene and other hygiene and healthcare products. The present invention further seeks to manage the distribution of said particles in a controlled manner, such that the distribution of said particles within the air-permeable, non-woven structure is predetermined by particle size, thereby to provide the most efficient mechanism for absorbing liquid waste as is feasible.

Hygiene products as referred to herein include, but are not limited to, infant diapers, adult incontinence products and feminine hygiene products. When in use by the consumer, each of these products is designed to manage and absorb liquids expelled from the user's body in the form of urine, liquid faeces and menstrual fluids individually or in combination.

Since the mid 1970's the inclusion of Super Absorbent Polymers (SAP) within infant diapers has been common practice. SAPs such as for example, sodium polyacrylate have usually been incorporated into the structure of infant diapers by blending the polymer particles into fluff pulp, to form an absorbent core of the diaper. Similar structures are also incorporated within adult incontinence and feminine hygiene products. Since the SAPs are only blended within the fluff pulp core it is necessary to take steps to stop the SAP particles from leaching out of the fluff pulp core and making contact with the skin of, for example, an infant wearer of the diaper. Although SAPs are considered harmless to human skin, it is not desirable to contaminate an infant's skin with SAP particles.

WO 2013/152809 A1 and WO 2013/153235 A1 relate to "unitary absorbent structures comprising an absorbent core and/or an acquisition and dispersion layer for absorbent particles".

Conventional methods for preventing SAP from leaching out of the fluff pulp diaper core include attaching lightweight layers of additional non-woven fabric and or polymer film onto both the surface and the edges of the fluff pulp core. Within the hygiene industry, this is often referred to as core wrap. This method of preventing SAP from leaching out of the fluff pulp core has been shown to be fallible and it is not uncommon for small amounts of SAP to leach out of the fluff pulp core and onto the infant or other wearer's skin.

It would therefore be desirable to eliminate fluff pulp entirely from the core of all hygiene products including infant diapers, adult incontinence products and feminine hygiene products thereby reducing the mass and thickness of the manufactured products whilst maintaining the ability of the products to absorb the required volumes of liquid waste whether it is urine, liquid faeces or menstrual fluids or any combination of these liquids whilst at the same time retaining the SAP particles fully within the core structure of the product.

The present invention is as defined in the claims. This invention and other aspects of the present disclosure may be more fully understood by reference to the following description. The present invention provides a method for incorporating absorbent particles within hygiene products in such a way that the particles are distributed within the air-permeable, non-woven structure of the product in a controlled and pre-determined manner such that the particles are able to absorb liquid waste in the most efficient manner feasible whilst also being fully retained within the structure of the product such that the particles are unable to leach out onto the wearer's skin.

According to the present invention there is provided a method for dissipating and entrapping absorbent particles within air permeable structures, for use in the construction of absorbent articles, said method comprising the steps of:
(i) constructing an air-permeable, non-woven structure comprising at least first, second and third layers of non-woven fabric, each said numbered layer (n) being bonded, manufactured onto or otherwise joined to each subsequently numbered layer (n+1), and wherein fibres in each said layer are arranged so as to define void spaces therebetween of pre-determined size, corresponding to a given absorbent particle size distribution range;
   and wherein the void spaces in each said numbered layer (n), are of smaller size than the void spaces in each subsequently numbered layer (n+1);
(ii) dispersing, by controllable mechanical means, absorbent particles onto an external surface of the highest numbered layer of said air-permeable, non-woven structure formed in step (i), said absorbent particles having a pre-determined particle size distribution range;
(iii) dissipating said dispersed absorbent particles within the air-permeable, non-woven structure by applying an external energy source acting upon the absorbent particles in a direction substantially normal to the plane of the external surface of the air-permeable, non-woven structure wherein the external energy source is an ultra-sonic vibration source.

In certain embodiments of the present invention, the method may comprise the additional further steps of:
(iv) subjecting at least one layer of the air-permeable, non-woven structure to an external heat source, and subsequently effecting or allowing cooling of said at least one layer, so as to entrap said absorbent particles within said air-permeable, non-woven structure; and/or
(v) welding, bonding or otherwise attaching a further layer to the external surface of the highest numbered layer of said air-permeable, non-woven structure incorporating said dissipated absorbent particles.

The first layer, as referred to in step (i), should be a hydrophilic or hydrophobic fibre layer constructed such that the spaces or voids between the individual fibres that constitute the first layer are too small for a first size of the particle size distribution (PSD) of a given grade of the absorbent particle types to be incorporated within the air-permeable, non-woven structure to pass through. Within the hygiene industry this layer is often referred to as the acquisition and distribution layer (ADL).

The second layer, attached, manufactured onto or otherwise fixed to the first layer, should comprise a second hydrophilic or hydrophobic fibre layer such that said first size of given grade of absorbent particles may pass into the spaces and or voids between the individual fibres that constitute the second layer but the same particles are unable to pass into or through the spaces or voids in the first described layer or ADL.

The third layer, attached, manufactured onto or otherwise fixed to the second layer, should comprise a third hydrophilic or hydrophobic fibre layer such that a second size of a given grade of absorbent particles may pass into the spaces and or voids between the individual fibres that constitute the third layer but the same size of particles are unable to pass into the spaces or voids in the second described layer.

Any number of additional layers may be added to the structure depending upon the particle size distribution of the SAP being used within the end product under manufacture and the pre-determined distribution of SAP through the cross-section of the air-permeable, non-woven structure according to the absorbency performance required.

Step (ii) involves dispersing the absorbent particles onto the surface of the uppermost of the air-permeable non-woven structure by a controllable mechanical means such as precision scatter coating, whereby the particles are mechanically distributed onto the surface via a rotary screen. In scatter coating, the particles to be dispersed are usually conveyed to a rotary screen through a closed particle feeding tube by a worm drive device where the particles flow onto a doctor blade which pushes the particles through the holes in a purpose designed screen directly onto the surface of the target air-permeable structure. Other types of scatter coating mechanisms are also suitable.

An alternative method of dispersing the absorbent particles onto the uppermost surface of the structure is by powder spraying, whereby the absorbent particles are accelerated though an orifice, usually with the assistance of compressed air. By controlling the mechanical design of the orifice and the air pressure provided, it is possible accurately to meter a specific mass of particles through the orifice in a given period of time, thereby facilitating accurate dispersion of said particles and creating either pre-determined patterns of dispersion or full width dispersion of absorbent particles.

A further alternative method of dispersing the absorbent particles onto the uppermost surface of the structure is with the implementation of a vibrating particle feeder system. Vibrating feeders systems may be supplied with either electromagnetic or vibrator motor drives.

Electromagnetic drive feeders may be used where the delivery flow of particles requires frequent adjustment, or is subject to constant stop/start cycles, as would be the case where intermittent dispersion patterns are required on the surface of the substrate onto which the particles are to be dispersed.

Using one of aforementioned methods or any other method of controlled dispersion, the particles may be dispersed across the entire surface of the air permeable structure, or only across selected, pre-determined areas of the air permeable structure depending upon the design requirements of the end manufactured product.

Although the previously described methods of dispersing the particles onto the air permeable structure are relatively accurate, it is inevitable that some areas of the structure will initially have a higher areal density of particles on the surface of the air-permeable, non-woven structure than other areas due to the localised agglomeration of the particles and/or the motion of either the dispersal system or the motion of the air permeable structure onto which the particles have been dispersed. In-process airflow due to the motion of the air-permeable structure during the dispersal phase may also result in localised agglomeration of the dispersed particles.

Depending upon the specific particle size of the absorbent particles used and the specific type and construction of the uppermost layer of the air-permeable, non-woven structure, some of the particles will become entrained within the structure by falling through the uppermost layer at the surface as a result of gravity.

Other particles may come to rest partially entrained within the uppermost layer and partially or wholly above the next layer down and so on, depending upon the number of individual layers that make up the air permeable structure, the specific construction of those individual layers and the PSD of the particular grade of SAP being dispersed.

Preferably, the absorbent particles have a mean particle size of between >10µm and <3000µm. In preferred embodiments, the mean particle size of the absorbent particles is in the range of between 25µm to 2500µm, more preferably 50µm to 1500µm, and most preferably 75µm to 1000µm. In an alternative embodiment, the mean particle size of the absorbent particles is preferably in the range of between 40µm to 900µm.

A number of alternative technologies are suitable for dissipating the particles throughout the layered construction of the air permeable substrate in step (iii).

In the claimed invention, dissipating the particles is carried out by applying an external energy source acting upon the absorbent particles in a direction substantially normal to the plane of the external surface of the air-permeable, non-woven structure, wherein the external energy source is an ultra-sonic vibration source.

In this method of dissipating the dispersed particles, the matrix is subject to a high frequency vibration source such as that provided by ultra-sonic vibrating sonotrode, whilst the particles are substantially in intimate contact with the uppermost layer onto which the particles have been dispersed.

For comparison, alternative methods of dissipating not belonging to the invention are as follows:
A first alternative method of dissipating the dispersed particles is to subject the whole matrix of dispersed particles and air-permeable, non-woven structure to an externally applied vibration energy (VE) of a frequency between 1Hz and 250Hz, and with an amplitude of between 0.1mm to 5mm. In practice, such vibration energy is transmitted to the matrix via either an electromagnetic or vibrator motor drive connected to a plate system over which the matrix is transported whilst the vibration energy is applied.

The primary effect of subjecting the particles to the VE is to de-agglomerate the particles whilst at the same time agitating the particles so that the particles become located substantially below the level of the uppermost surface of the air-permeable structure onto which the particles were first dispersed. Each particle size will thus come to rest generally within a layer of the structure designed to accommodate that given particle size whilst preventing larger particles from passing through a given layer into any of the lower layers.

A second alternative method of dissipating the dispersed particles is to subject the matrix to an atmospheric pressure, alternating electrical field (AEF) of between 1kV and 250kV at a frequency of between 1Hz to 250Hz whilst the particles are substantially in intimate contact with the uppermost layer.

The primary effect of subjecting the particles to the AEF is to de-agglomerate the particles, whilst at the same time agitating the particles so that they become located substantially below the level of the uppermost surface of the structure onto which the particles were first dispersed. Each particle size will thus come to rest generally within a layer of the structure designed to accommodate that given particle size whilst preventing larger particles from passing through a given layer into any of the lower layers.

The effect of the combined pre-determined construction of the air-permeable, non-woven structure together with the specific grade and therefore PSD of the SAP selected will result in the particles becoming distributed throughout a cross-section of the air-permeable structure according to the specific size of a given amount of particles and the void space between the fibres that constitute each individual layer of the air permeable structure.

Simply described, the larger particle sizes will reside in the upper layers of the structure and the smaller particle sizes will reside in the lower layers of the structure but no particles will be able to pass through the entire structure.

Preferably, the frequency of the applied VE is in the range of 1Hz to 250Hz. Preferably the frequency of the applied VE is in the range of 5Hz to 200Hz, preferably 10Hz to 150Hz, more preferably 20Hz to 100Hz, still more preferably 30Hz to 80Hz, further preferably 40Hz to 60Hz and most preferably 45Hz to 55Hz.

Preferably, the amplitude of the applied VE is in the range of 0.1mm to 5mm. Preferably the amplitude of the applied VE is 0.2mm to 4mm, preferably 0.3mm to 3mm, more preferably 0.4mm to 2mm and most preferably 0.5mm to 1.5mm.

Preferably, the voltage applied to generate the AEF is in the range of 1kV to 1000kV. Preferably the voltage applied to generate the AEF is in the range of 10kV to 1000kV, preferably 20kV to 500kV, more preferably 30kV to 200kV, still more preferably 40kV to 100kV and most preferably 50kV to 75kV. Preferably the voltage applied to generate the AEF is in the range of 10kV to 50kV.

Preferably, the power supply of the AEF is in the frequency range of between 1Hz to 100Hz. Preferably the power supply of the AEF is in the frequency range of between 1Hz to 250Hz, more preferably 10Hz to 100Hz, still more preferably 20Hz to 60kHz and most preferably between 50Hz to 60Hz.

The AEF may be configured with sinusoidal or square-wave alternating currents between utility frequencies of 50Hz to 60Hz or with pulsed wave forms depending upon the variable conditions of the absorbent particle size, the rate of dispersion of the particles onto the surface of the air-permeable non-woven structure and the construction, density and thickness of the structure.

A third alternative method of dissipating the dispersed particles is to subject the matrix to a vacuum process, whilst the particles are substantially in intimate contact with the uppermost layer onto which the particles have been dispersed, the vacuum being applied from beneath the lowermost layer to draw the dispersed particles into the entire layered structure of the matrix.

An optional fourth step is to consolidate the particles within the layers of the air-permeable substrate, if required. In some cases, it will not be required or desired, due to the specific construction of the end product, to consolidate the particles within the air permeable structure. For example, if the SAP is dispersed and then dissipated throughout the air-permeable structure in line with the end product manufacturing process of an infant diaper then a next step of the production process would be to attach a polymer film to the uppermost layer of the structure. This polymer sheet is often referred to within the diaper manufacturing industry as the back sheet.

If however, the SAP dispersed and dissipated air permeable structures are to be incorporated into the end product in a second manufacturing process, then it may become necessary to consolidate the SAP particles within the air-permeable structure so that the particles do not become dislodged during transport to a second production site or during the secondary end product manufacturing phase.

There are many ways of consolidating the particles within air-permeable structure layers depending upon the specific construction and the specific materials that make up the air-permeable structure and the type and size of absorbent particles that are dispersed and dissipated within the matrix.

One example of a method of consolidating the SAP within the air permeable structure is to construct the uppermost layer of the structure to include fibres that can be made to extend and shrink at differential rates in length when subjected to an external heating source. Said fibres thus crimp and lock adjacent fibres together, having the effect of reducing the spaces and or voids between the fibres thereby to prohibit previously dissipated particles from exiting the air-permeable structure, whilst retaining the ability of the matrix to acquire, distribute and absorb liquid waste in an efficient manner.

A second example of a method of consolidating the SAP within the air-permeable structure is to attach a further layer of non-woven fabric to the surface of the uppermost layer of the structure after the entire matrix has been subjected to a dissipation process, thereby mechanically prohibiting the now entrained SAP from exiting the air permeable structure. The further layer of non-woven fabric may have hydrophobic or hydrophilic properties and may be attached to the uppermost layer by welding, gluing, bonding, stitching or other suitable means compatible with the desired properties of the end product to be manufactured.

A third example of a method of consolidating the SAP within the air-permeable structure is to incorporate lower melt temperature fibres together with higher melt temperature fibres within the uppermost layer. After the entire matrix has been subjected to a dissipation process, an external heat source may be applied to the uppermost layer of the structure so that the lower melt temperature fibres soften and become tacky thereby adhering to the surface of the SAP particles located in the uppermost layer. Upon cooling, the SAP in the uppermost layer will become bonded to the fibres that form the layer, and create a physical barrier to those smaller particles already entrained within the lower levels of the structure and prevent the egress of said particles from within the air-permeable structure.

A fourth example of a method of consolidating the SAP within the air permeable structure is to incorporate a binding agent or agents within the air-permeable structure during the manufacture of the structure itself. After the SAP has been dispersed on the uppermost surface and been subjected to an AEF to de-agglomerate and dissipate the SAP, the entire matrix is subjected to an external heat source to activate the binder or binders within the air-permeable structure, thereby causing the binder or binders to lock the SAP within the structure.

In some circumstances, it may also be desirable to ensure that no dissipated SAP within the air-permeable structure can egress the structure in post-dissipation processes such as slitting.

The method of the present invention may also include further steps of cutting the air-permeable non-woven structure, and subsequently sealing the edges thereof Suitable methods for such sealing include, but are not limited to: ultra-sonic welding, radio frequency welding, heat welding, impulse welding, stitching, binding, bonding and other similar processes.

In order that the present invention may be fully understood, preferred embodiments thereof will now be described in detail, though only by way of example, with reference to the accompanying drawings in which:
Figure 1 is a cross-sectional view of an air-permeable, non-woven structure as formed in step (i) of the method of the present invention;
Figure 2 is a cross-sectional view of the air-permeable, non-woven structure of Figure 1, having absorbent particles dispersed thereon, as per step (ii) of the method of the present invention;
Figure 3 is a cross-sectional view of the air-permeable, non-woven structure of Figure 2, following dissipation of the absorbent particles, as per step (iii) of the method of the present invention;
Figure 4 is a cross-sectional view of the air-permeable, non-woven structure of Figure 3, following consolidation of the absorbent particle and non-woven matrix, as per step (iv) of the method of the present invention;
Figure 5 is a top view of an absorbent particle and non-woven matrix having absorbent particles arranged in a pre-determined pattern;
Figure 6 is a top view of an absorbent article, having been cut from the matrix of Figure 5;
Figure 7 is an illustration of a process layout for performing steps (ii) and (iii) of a first embodiment of the method of the present invention;
Figure 8 is an illustration of a comparative process not belonging to the invetion.
Figure 9 is an illustration of a comparative process not belonging to the invention. and
Figure 10 is an illustration of a comparative process not belonging to the invetion.

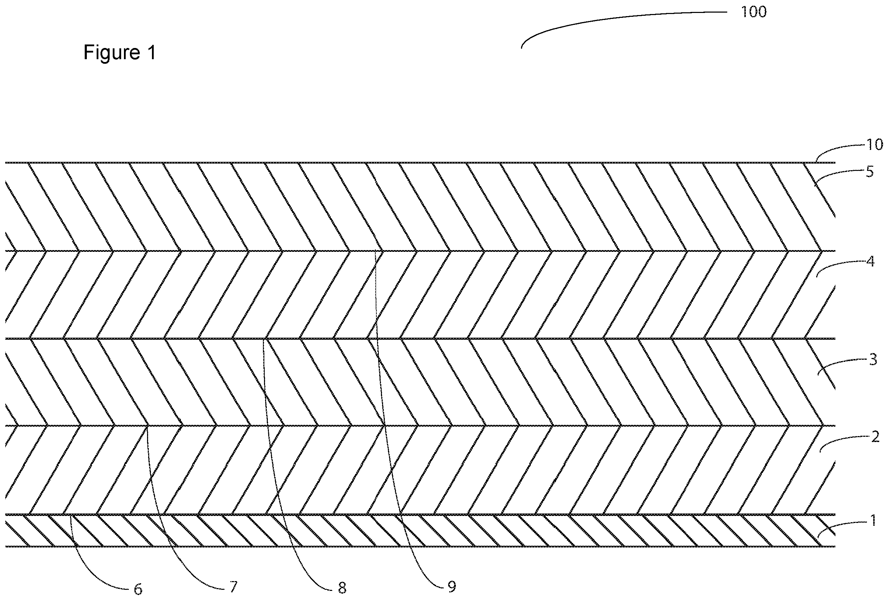

Referring first to Figure 1, there is shown an air-permeable, non-woven structure, generally indicated 100, as formed in step (i) of the method of the present invention. A first layer 1 comprised of non-woven fibres is bonded, manufactured onto or otherwise attached to a second layer 2 at interface 6; a third layer 3 is bonded, manufactured onto or otherwise attached to the second layer 2 at interface 7; a fourth layer 4 is bonded, manufactured onto or otherwise attached to the third layer 3 at interface 8; and a fifth layer 5 is bonded, manufactured onto or otherwise attached to the fourth layer 4 at interface 9. The surface 10 of the fifth layer 5 in this embodiment is the uppermost surface. The fifth layer 5 comprises bi-component fibres in which the two components have differential melting temperatures.

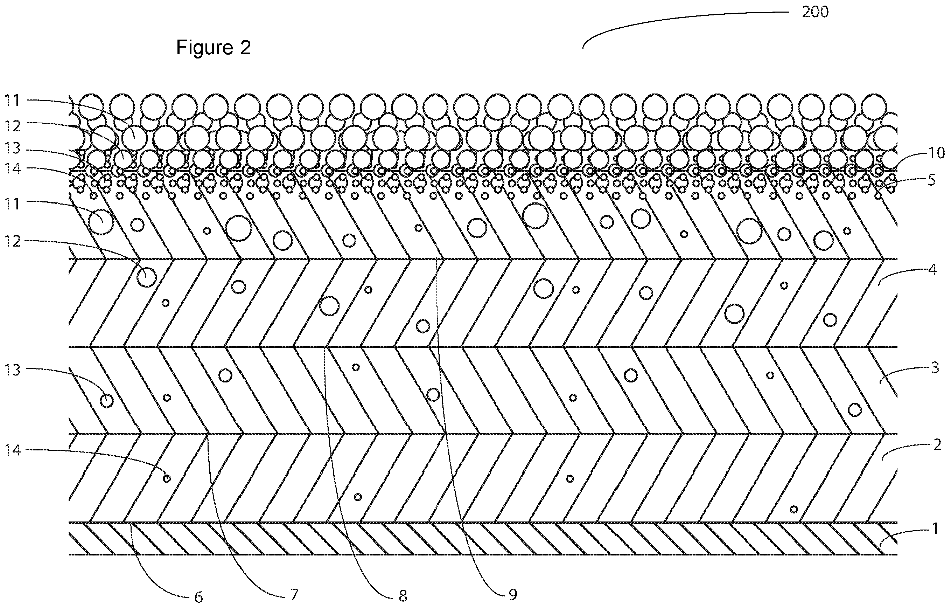

Referring now to Figure 2, there is shown the air-permeable, non-woven structure described above with reference to Figure 1, now generally indicated 200. Absorbent particles 11, 12, 13 and 14 of varying particle sizes, have been dispersed onto the uppermost surface 10 of the fifth layer 5, as per step (ii) of the method of the present invention. Due to the relatively open structure of the surface of the fifth layer 5, some of the particles 11, 12, 13 and 14 have passed into the subsequent layers 2, 3, 4 and 5. Some of the absorbent particles have thus come to rest in layers where the void space within that specific layer allows free passage of the absorbent particles. Other particles 11, 12, 13 and 14 have come to rest partially above and partially below the surface of the fifth layer 5 such that absorbent particles 11 are prevented from passing into the fourth layer 4 at interface 9 due to the restrictive spaces between the fibres that constitute the fourth layer 4; whilst absorbent particles 12 are prevented from passing into the third layer 3 at interface 8 due to the restrictive spaces between the fibres that constitute the third layer 3; absorbent particles 13 are prevented from passing into the second layer 2 at interface 7 due to the restrictive spaces between the fibres that constitute the second layer 2; whilst the restrictive void spaces between the fibres that constitute the first layer 1 are too small to allow the passage of any of the absorbent particles, irrespective of their size.

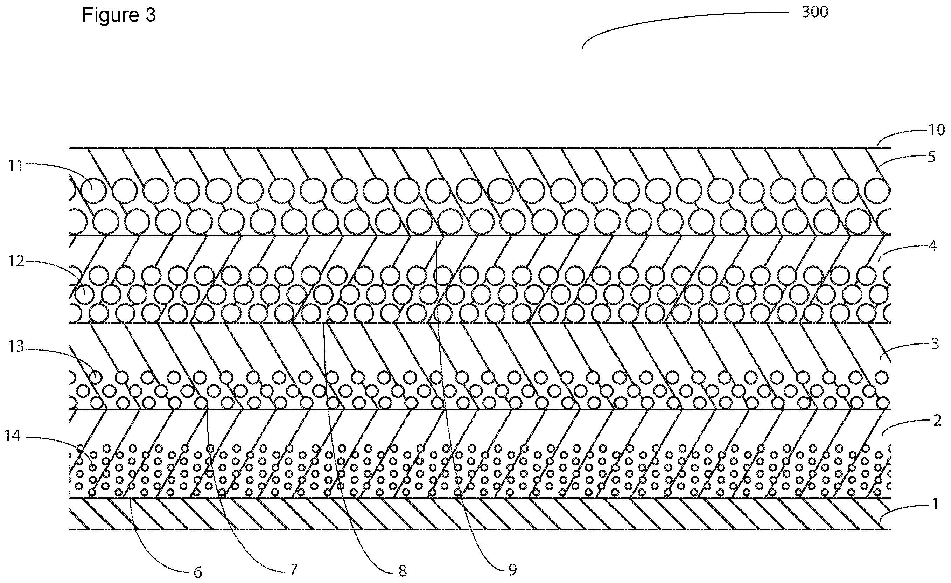

Referring now to Figure 3, there is shown the air-permeable, non-woven structure described above with reference to Figures 1 and 2, now generally indicated 300. The structure 300, and the dispersed absorbent particles 11, 12, 13 and 14 have now been exposed to a particle dissipation process as per step (iii) of the method of the present invention. The absorbent particles 11, 12, 13 and 14 have been de-agglomerated and as a result of the effect of the dissipation process have become located substantially below the uppermost surface 10 of the fifth layer 5 and have become substantially dispersed throughout the structure according to both the void space size within each of the individual layers 2, 3, 4 and 5 and the specific particle size distribution profile of the absorbent being dispersed. Particles 11, 12, 13 and 14 have come to rest substantially below the surface of the fifth layer 5; absorbent particles 11 are prevented from passing into the fourth layer 4 at interface 9 due to the restrictive spaces between the fibres that constitute the fourth layer 4; absorbent particles 12 are prevented from passing into the third layer 3 at interface 8 due to the restrictive spaces between the fibres that constitute the third layer 3; absorbent particles 13 are prevented from passing into the second layer 2 at interface 7 due to the restrictive spaces between the fibres that constitute the second layer 2; whilst the restrictive void spaces between the fibres that constitute layer 1 are too small to allow the passage of any of the absorbent particles, irrespective of their size.

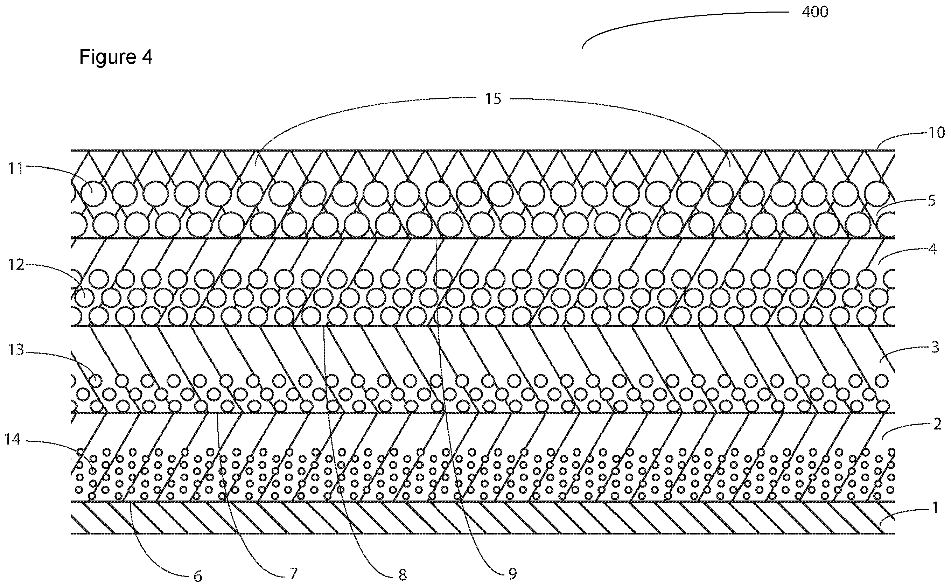

Referring now to Figure 4, there is shown the air-permeable, non-woven structure described above with reference to Figures 1 to 3, now generally indicated 400, following the performance of a consolidation process, as per optional step (iv) of the method of the present invention. The consolidation process has been applied to the uppermost (fifth) layer 5 such that now dissipated particles 11, 12, 13 and 14 are prevented from exiting the air-permeable structure via the uppermost (fifth) layer 5 due to the consolidation of fibres in region 15 of the fifth layer 5.

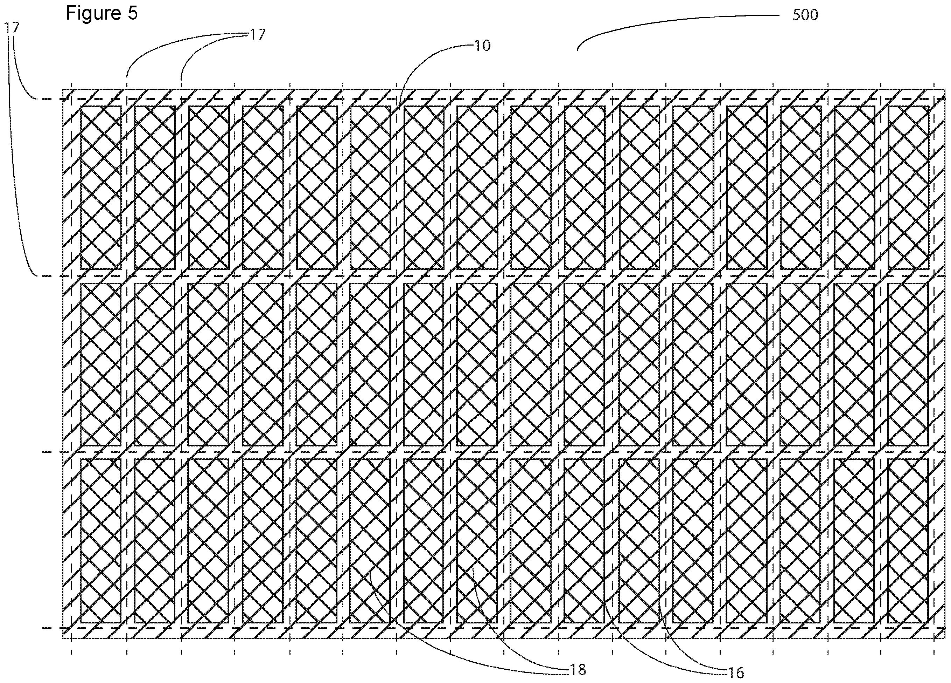

Referring now to Figure 5, there is shown an absorbent particle and non-woven matrix, generally indicated 500, emerging from method step (iv), as described above with reference to Figure 4. In this embodiment, the uppermost surface 10 of the fifth layer 5 has had absorbent particles dispersed upon it in a pre-determined pattern or shape 16. Following exposure to a suitable dissipation process (iii) followed by consolidation (iv) of the particles, an portion 18 of the matrix 500 may be cut or otherwise extracted from the air permeable non-woven matrix 500 along path 17, such that the path of the cut line 17 is larger in size than the pre-determined pattern or shape 16.

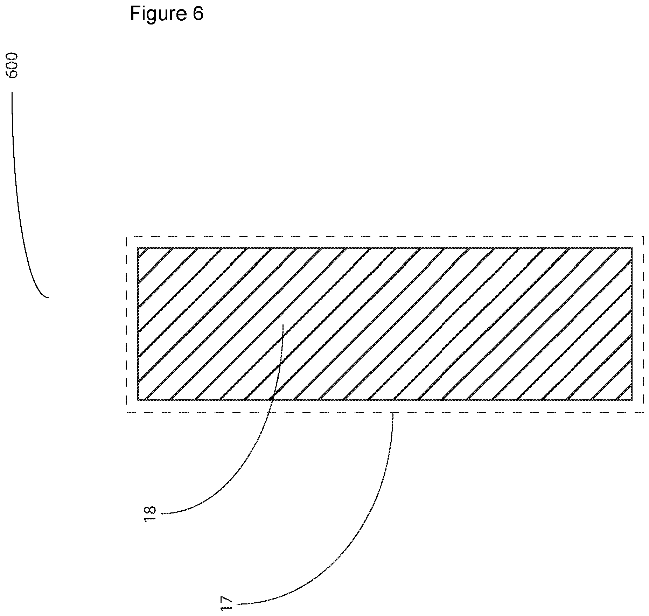

Referring now to Figure 6, there is shown an absorbent article generally indicated 600, following cutting or otherwise extracting the portion 18 of the particle and non-woven matrix 500 along path 17, as described above with reference to Figure 5.

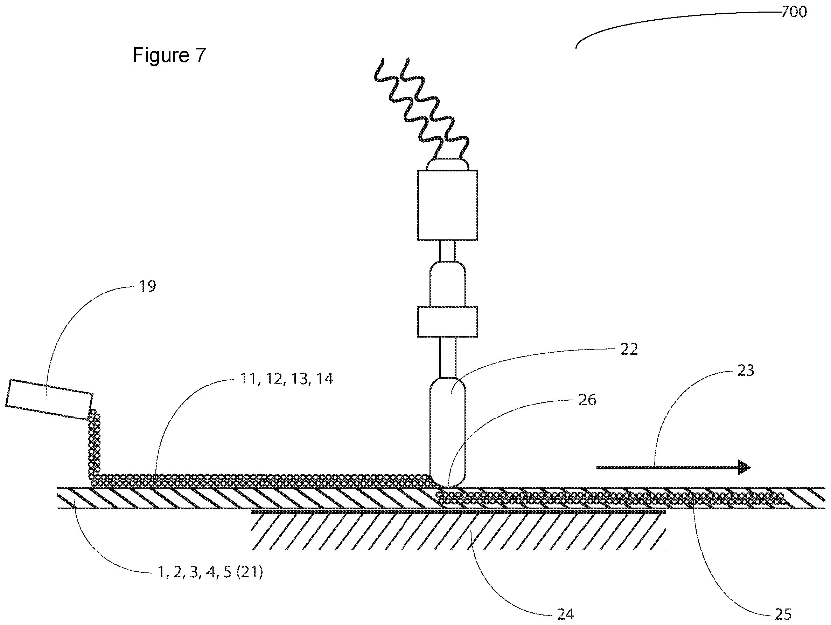

Referring now to Figure 7, there is shown a process layout 700 for performing steps (ii) and (iii) of a first embodiment of the method of the present invention. Absorbent particles 11, 12, 13 and 14 are dispersed, as per step (ii), onto the surface of an air-permeable, non-woven structure 21, comprising layers 1, 2, 3, 4 and 5, by means of a controlled scattering device 19. The non-woven structure 21 is moved in the direction of arrow 23 across a base plate 24 whilst simultaneously a substantially vertically applied ultra-sonic force is applied, as per step (iii), at the interface 26 of an ultra-sonic device 22 causing the particles 11, 12, 13 and 14 to become entrained within the non-woven layers 25.

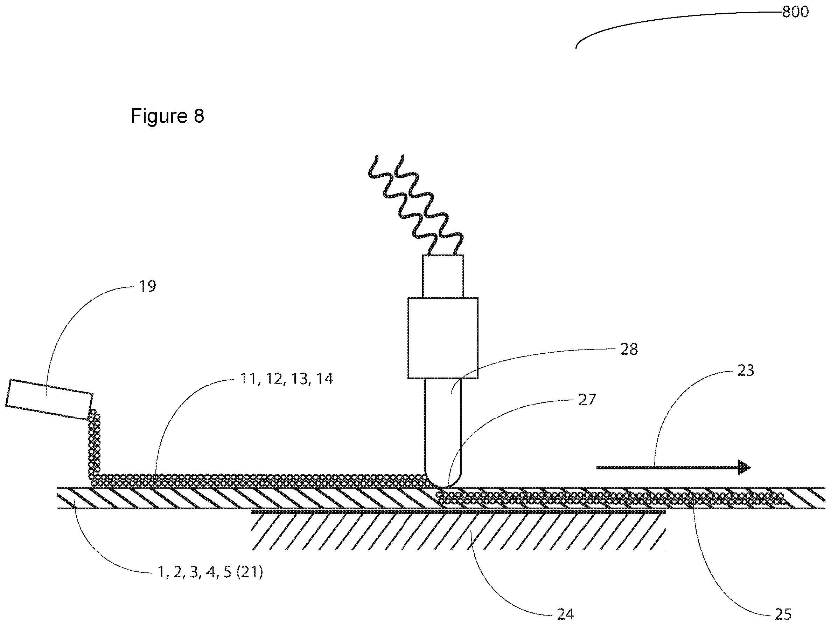

Referring now to Figure 8, there is shown a comparative process layout 800 not belonging to the invetion. Absorbent particles 11, 12, 13 and 14 are dispersed, as per step (ii) onto the surface of an air-permeable, non-woven structure 21, comprising layers 1, 2, 3, 4 and 5 by means of a controlled scattering device 19. The non-woven layer 21 is moved in the direction of arrow 23 across a base plate 24 whilst simultaneously a substantially vertically applied low frequency vibration in the range of 10Hz to 200Hz is applied, as per step (iii), at the interface 27 of a low frequency vibration device 28 causing the particles 11, 12, 13 and 14 to become entrained within the non-woven layers 25.

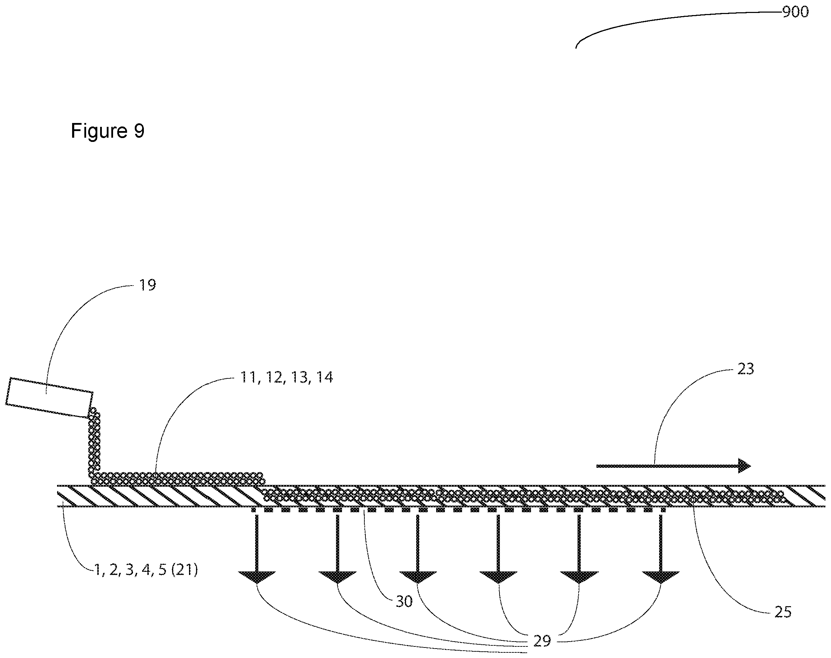

Referring now to Figure 9, there is shown a comparative process layout 900 not belonging to the invetion. Absorbent particles 11, 12, 13 and 14 are dispersed, as per step (ii), onto the surface of an air-permeable, non-woven structure 21, comprising layers 1, 2, 3, 4 and 5, by means of a controlled scattering device 19. The non-woven structure 21 is moved in the direction of arrow 23 across a perforated plate or membrane 30 whilst simultaneously a substantially vertically applied vacuum force 29 is applied, as per step (iii), from beneath the perforated plate or membrane 30 causing the particles 11, 12, 13 and 14 to become entrained within the non-woven layers 25.

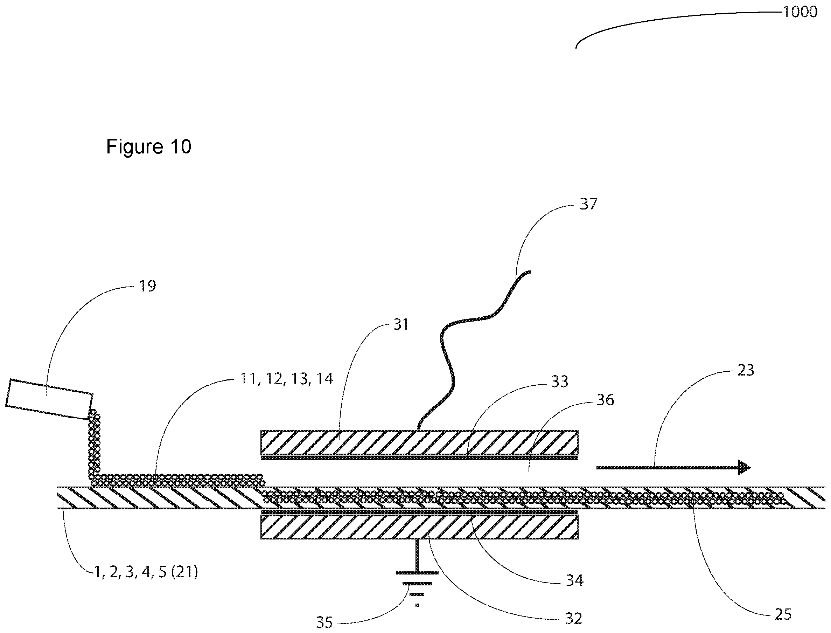

Referring finally to Figure 10, there is shown a comparative process layout 1000 not belonging to the invention. Absorbent particles 11, 12, 13 and 14 are dispersed, as per step (ii) onto the surface of an air-permeable, non-woven an air-permeable, layers 1, 2, 3, 4 and 5, by means of a controlled scattering device 19. The non-woven structure 21 is moved in the direction of arrow 23 between an upper electrode device 31 and a lower electrode device 32, each electrode device having a dielectric plate 33 and 34 located between said electrode devices 31 and 32 and the target particles 11, 12, 13 and 14 together with the non-woven structure 21. The upper electrode device 31 is connected to a high voltage, alternating current generator via cable 37 whilst the lower electrode device 32 is connected to earth 35. As the dispersed powder 11, 12, 13 and 14 is transported into the zone 36 between the two dielectric plates 33 and 34, the energy field generated between the upper electrode 31 and lower electrode 32 causes the powder to become excited and vibrate in a plane substantially normal to the plane of the dielectric plates 33 and 34 and become dissipated, as per step (iii), within the non-woven layers 25.

The method according to the present invention will now be further described by way of the following example and comparative examples:

### Comparative Example 1

A non-woven fabric structure was manufactured comprising five individual layers of varying void space dimensions between individual and groups of fibres. Each of the four lower layers were manufactured using a blend of polypropylene, polyethylene and polyester fibres in the ratio of 20%, 40% and 40% respectively.

The construction of the entire non-woven structure was such that the lowermost layer or first layer (ADL) would not permit the entry into or passage through of any Super Absorbent Polymer (SAP) particles of less than 10µm in minimum diameter. Effectively, this layer was manufactured to act as a physical barrier to the passage of any of the SAP to be processed within this entire example.

The next layer or second layer was manufactured to permit the entry of SAP particles in the range of 10µm to 150µm but not to allow the entry of or passage through of SAP particles of particle size greater than 150µm.

The next or third layer was manufactured to permit the entry of SAP particles in the range 10µm to 400µm but not to permit the entry of or passage through of SAP of particle size greater than 400µm.

The next or fourth layer was manufactured to permit the entry of SAP particles in the range 10µm to 600µm but not to permit the entry or passage through of SAP of particle size greater than 600µm.

The next or fifth layer was manufactured to permit the entry of SAP particles in the range 10µm and above. The uppermost layer of the non-woven structure was manufactured from bi-component fibres in a side by side configuration from polyester and polyethylene.

The method of manufacture of the non-woven substrate as a whole was a combination of conventional random carding, air through and needle punch processes.

A sodium polyacrylate SAP of particle size distribution ranging between 40µm to 850µm commercially manufactured by a market leader in the hygiene materials supply market was mechanically dispersed onto the uppermost surface of the aforesaid high loft non-woven fabric at an areal dispersion rate of 325 gsm (gm⁻²) which in practice would equate to an amount of 13g in a typical infant diaper core.

The SAP and non-woven fabric structure was then subjected to an external vibrating energy source of frequency 50Hz and amplitude 1.5mm to dissipate the SAP particles within the structure of the non-woven. The particles were caused to come to rest within the structure in a gradient manner according to the specific PSD of the SAP particles and the void space at a given location within the non-woven fabric structure.

Following the dissipation process, the uppermost layer of the non-woven fabric structure was then subjected to an external heat source provided by infra-red heating lamps, to cause the bi-component fibres in the uppermost layer of the structure to crimp as a result of differential expansion of each component of the bi-component fibres.

The entire non-woven fabric structure and SAP matrix was then allowed to cool. After cooling it was found that the SAP was fully contained within the non-woven structure.

### Comparative Example 2

A non-woven fabric structure was manufactured comprising five individual layers of varying void space dimensions between individual and groups of fibres. Each of the four lower layers were manufactured using a blend of polypropylene, polyethylene and polyester fibres in the ratio of 20%, 40% and 40% respectively.

The construction of the non-woven structure was such that the lowermost layer or first layer (ADL) would not permit the entry into or passage through of any Super Absorbent Polymer particles (SAP) of less than 10µm in minimum diameter. Effectively, this layer was manufactured to act as a physical barrier to the passage of any of the SAP to be processed within this example.

The next layer or second layer was manufactured to permit the entry of SAP particles in the range of 10µm to 150µm but not to allow the entry of or passage through of SAP of particle size greater than 150µm.

The next or third layer was manufactured to permit the entry of SAP particles in the range 10µm to 400µm but not to permit the entry of or passage through of SAP of particle size greater than 400µm.

The next or fourth layer was manufactured to permit the entry of SAP particles in the range 10µm to 600µm but not to permit the entry or passage through of SAP of particle size greater than 600µm.

The next or fifth layer was manufactured to permit the entry of SAP particles in the range 10µm and above. The uppermost layer of the non-woven structure was manufactured from bi-component fibres in a side by side configuration from polyester and polyethylene.

The method of manufacture of the non-woven substrate as a whole was a combination of conventional random carding, air through and needle punch processes.

A sodium polyacrylate SAP of particle size distribution ranging between 40µm to 850µm commercially manufactured by a market leader in the hygiene materials supply market was mechanically dispersed onto the uppermost surface of the aforesaid high loft non-woven fabric at an areal dispersion rate of 325 gsm (gm⁻²) which in practice would equate to an amount of 13g in a typical infant diaper core.

The SAP and non-woven fabric structure was then subjected to an alternating voltage energy field (AVEF) of 25kV and of frequency 50Hz between two opposed electrode plates placed 10mm apart along their longitudinal axis to excite the SAP particles such that the particles were made to vibrate in a direction normal to the opposed surfaces of the electrode plates, the vibration energy being sufficient to dissipate the SAP within the non-woven fabric structure such that no SAP remained on the uppermost surface of the non-woven fabric structure.

Following the dissipation process the entire non-woven structure and SAP matrix was subject to an air-through heating process such that the lower melting temperature component of the bi-component fibres that constitute the uppermost layer of the structure became soft and tacky such as to bond to adjacent individual and groups of fibres thereby entrapping the SAP dissipated within the uppermost layer and creating a physical barrier to those SAP particles dissipated within the adjacent and lower layer from passing out through the uppermost layer upon the entire matrix being subjected to agitation.

Upon cooling, the lower melting temperature component of the bi-component fibres in the uppermost layer of the structure retained their solid state now bonded to adjacent individual and groups of fibres.

### Comparative Example 3

A non-woven fabric structure was manufactured comprising five individual layers of varying void space dimensions between individual and groups of fibres. Each of the four lower layers were manufactured using a blend of polypropylene, polyethylene and polyester fibres in the ratio of 20%, 40% and 40% respectively.

The construction of the entire non-woven structure was such that the lower most layer or first layer (ADL) would not permit the entry into or passage through of any Super Absorbent Polymer particles (SAP) of less than 10µm in minimum diameter. Effectively, this layer was manufactured to act as a physical barrier to the passage of any of the SAP to be processed within this example.

The next layer or second layer was manufactured to permit the entry into of SAP particles in the range of 10µm to 150µm but not to allow the entry of or passage through of SAP of particle size greater than 150µm.

The next or third layer was manufactured to permit the entry of SAP particles in the range 10µm to 400µm but not to permit the entry of or passage through of SAP of particle size greater than 400µm.

The next or fourth layer was manufactured to permit the entry of SAP particles in the range 10µm to 600µm but not to permit the entry or passage through of SAP of particle size greater than 600µm.

The next or fifth layer was manufactured to permit the entry of SAP particles in the range 10µm and above. The uppermost layer of the non-woven structure was manufactured from bi-component fibres in a side by side configuration from polyester and polyethylene.

The method of manufacture of the non-woven substrate as a whole was a combination of conventional random carding, air through and needle punch processes.

A sodium polyacrylate SAP of particle size distribution ranging between 40µm to 850µm commercially manufactured by a market leader in the hygiene materials supply market was mechanically dispersed onto the uppermost surface of the aforesaid high loft non-woven fabric structure at an areal dispersion rate of 325 gsm (gm⁻²) which in practice would equate to an amount of 13g in a typical infant diaper core.

The SAP and non-woven fabric structure was then subjected to a vacuum, applied from below the lowermost surface of the structure of greater than 1 bar in pressure such that the SAP particles dispersed upon the uppermost layer of the structure were drawn into the structure with the SAP particles coming to rest within a specific given layer dependent upon the given diameter of the SAP particle in question.

Following the dissipation process as a result of the applied vacuum, a sixth layer of spun bonded polypropylene non-woven fabric of areal weight of 8 gsm (gm⁻²) was then attached to the uppermost surface of the SAP and non-woven fabric matrix by means of intermittent ultra-sonic welding such that the SAP particles now dissipated within the non-woven fabric structure were prohibited from egress via the uppermost layer of the structure.

### Comparative Example 4

A non-woven fabric structure was manufactured comprising five individual layers of varying void space dimensions between individual and groups of fibres. Each of the four lower layers were manufactured using a blend of polypropylene, polyethylene and polyester fibres in the ratio of 20%, 40% and 40% respectively.

The construction of the entire non-woven structure was such that the lower most layer or first layer (ADL) would not permit the entry into or passage through of any Super Absorbent Polymer particles (SAP) of less than 10µm in minimum diameter. Effectively, this layer was manufactured to act as a physical barrier to the passage of any of the SAP to be processed within this example.

The next layer or second layer was manufactured to permit the entry into of SAP particles in the range of 10µm to 150µm but would not allow the entry of or passage through of SAP of particle size greater than 150µm.

The next or third layer was manufactured to permit the entry of SAP particles in the range 10µm to 400µm but would not permit the entry of or passage through of SAP of particle size greater than 400µm.

The next or fourth layer was manufactured to permit the entry of SAP particles in the range 10µm to 600µm but would not permit the entry or passage through of SAP of particle size greater than 600µm.

The next or fifth layer was manufactured to permit the entry of SAP particles in the range 10µm and above. The uppermost layer of the non-woven structure was manufactured from bi-component fibres in a side by side configuration from polyester and polyethylene.

The method of manufacture of the non-woven substrate as a whole was a combination of conventional random carding, air through and needle punch processes.

A sodium polyacrylate SAP of particle size distribution ranging between 40µm to 850µm) commercially manufactured by a market leader in the hygiene materials supply market was mechanically dispersed onto the uppermost surface of the aforesaid high loft non-woven fabric structure at an areal dispersion rate of 325 gsm (gm⁻²) which in practice would equate to an amount of 13g in a typical infant diaper core.

The SAP and non-woven fabric structure was then subjected to an external vibrating energy source of frequency 50Hz and amplitude 1.5mm to dissipate the SAP particles within the structure of the non-woven fabric structure. The particles were caused to come to rest within the substrate in a gradient manner according to the specific PSD of the SAP particles and the void space at a given location within the cross-section of the entire non-woven fabric structure.

Following the dissipation process, a polyethylene film, coated on one surface with a heat sensitive adhesive based on polyurethane chemistry, was bonded onto the uppermost surface of the uppermost layer such that the SAP particles now dissipated within the non-woven fabric structure were prohibited from egress via the uppermost layer of the substrate.

### Example 5

A non-woven fabric structure was manufactured comprising five individual layers of varying void space dimensions between individual and groups of fibres. Each of the four lower layers were manufactured using a blend of polypropylene, polyethylene and polyester fibres in the ratio of 20%, 40% and 40% respectively.

The construction of the entire non-woven structure was such that the lowermost layer or first layer (ADL) would not permit the entry into or passage through of any Super Absorbent Polymer particles (SAP) of less than 10µm in minimum diameter. Effectively, this layer was manufactured to act as a physical barrier to the passage of any of the SAP to be processed within this example.

The next layer or second layer was manufactured to permit the entry into of SAP particles in the range of 10µm to 150µm but not to allow the entry of or passage through of SAP of particle size greater than 150µm.

The next or third layer was manufactured to permit the entry of SAP particles in the range 10µm to 400µm but not to permit the entry of or passage through of SAP of particle size greater than 400µm.

The next or fourth layer was manufactured to permit the entry of SAP particles in the range 10µm to 600µm but not to permit the entry or passage through of SAP of particle size greater than 600µm.

The next or fifth layer was manufactured to permit the entry of SAP particles in the range 10µm and above. The uppermost layer of the non-woven structure was manufactured from bi-component fibres in a side by side configuration from polyester and polyethylene.

The method of manufacture of the non-woven substrate as a whole was a combination of conventional random carding, air through and needle punch processes. A sodium polyacrylate Super Absorbent Polymer (SAP) of particle size distribution ranging between 40µm to 850µm commercially manufactured by a market leader in the hygiene materials supply market was mechanically dispersed onto the uppermost surface of the aforesaid high loft non-woven fabric at an areal dispersion rate of 325 gsm (gm⁻²) which in practice would equate to an amount of 13g in a typical infant diaper core.

The SAP, now dispersed onto the uppermost surface of the non-woven fabric, was then subjected to an ultra-sonic energy source of 15kHz and with an amplitude of 90 microns via a suitably engineered sonotrode to excite the SAP particles such that the particles were made to vibrate in a direction normal to surface of the non-woven fabric structure, the vibration energy being sufficient to dissipate the SAP within the non-woven fabric structure such that no SAP remained on the uppermost surface of the non-woven fabric structure.

Following the dissipation process the non-woven structure and SAP matrix was subject to an external heating process such that the lower melting temperature component of the bi-component fibres that constitute the uppermost layer of the structure became soft and tacky such as to bond to adjacent individual and groups of fibres thereby entrapping the SAP dissipated within the uppermost layer and creating a physical barrier to those SAP particles dissipated within the adjacent and lower layers from passing out through the uppermost layer upon the entire matrix being subjected to agitation.

Upon cooling, the lower melting temperature component of the bi-component fibres in the uppermost layer of the structure retained its solid state now bonded to adjacent individual and groups of fibres.

## Claims

1. A method for dissipating and entrapping absorbent particles within air permeable structures, for use in the construction of absorbent articles, said method comprising the steps of:
(i) constructing an air-permeable, non-woven structure comprising at least first, second and third layers of non-woven fabric, each said numbered layer (n) being bonded, manufactured onto or otherwise joined to each subsequently numbered layer (n+1), and wherein fibres in each said layer are arranged so as to define void spaces therebetween of pre-determined size, corresponding to a given absorbent particle size distribution range;
and wherein the void spaces in each said numbered layer (n), are of smaller size than the void spaces in each subsequently numbered layer (n+1);
(ii) dispersing, by controllable mechanical means, absorbent particles onto an external surface of the highest numbered layer of said air-permeable, non-woven structure formed in step (i), said absorbent particles having a pre-determined particle size distribution range;
(iii) dissipating said dispersed absorbent particles within the air-permeable, non-woven structure by applying an external energy source acting upon the absorbent particles in a direction substantially normal to the plane of the external surface of the air-permeable, non-woven structure,
wherein the external energy source is an ultra-sonic vibration source.

2. A method as claimed in claim 1, wherein the ultra-sonic vibration source in step (iii) generates a frequency in the range of between 10kHz and 50kHz, and an amplitude in the range of between 5 microns (µm) and 500 microns (µm).

3. A method as claimed in any of the preceding claims wherein:
(a) the highest numbered layer of the air-permeable, non-woven structure comprises bi-component fibres, each said component having differing thermal expansion properties;
(b) each layer of the air- permeable, non-woven structure, with the exception of the first said layer, comprises bi-component fibres, each said component having differing thermal expansion properties; and/or
(c) at least one of the layers of the air-permeable, non-woven structure comprises a blend of at least two different fibre types having differing thermal expansion properties.

4. A method as claimed in claim 3, further comprising the step, after the dissipation step (iii), of:
(iv) subjecting at least one layer of the air-permeable, non-woven structure to an external heat source, and subsequently effecting or allowing cooling of said at least one layer, so as to entrap said absorbent particles within said air-permeable, non-woven structure.

5. A method as claimed in claim 4 wherein the highest numbered layer of the air-permeable, non-woven structure comprises bi-component fibres, each said component having differing thermal expansion properties,
wherein in step (iv), the highest numbered layer of the air-permeable, non-woven structure is subjected to an external heat source, such that said components in said bi-component fibres expand upon heating and contract upon cooling at differential rates, causing said fibres to curl or crimp, thereby entrapping said absorbent particles within said highest numbered layer.

6. A method as claimed in claim 4 wherein each layer of the air- permeable, non-woven structure, with the exception of the first said layer, comprises bi-component fibres, each said component having differing thermal expansion properties,
wherein in step (iv), all of the layers of the air-permeable, non-woven structure are subjected to an external heat source, such that said components in said bi-component fibres expand upon heating and contract upon cooling at differential rates, causing the fibres to curl or crimp, thereby entrapping said absorbent particles within said air-permeable, non-woven structure.

7. A method as claimed in claim 4 wherein at least one of the layers of the air-permeable, non-woven structure comprises a blend of at least two different fibre types having differing thermal expansion properties,
wherein in step (iv), all of the layers of the air-permeable, non-woven structure are subjected to an external heat source, such that the fibres having the lowest melting temperature soften and become tacky so as to adhere to adjacent absorbent particles thereby entrapping said absorbent particles within said air-permeable, non-woven structure.

8. A method as claimed in any of the preceding claims, further comprising the step of:
(v) welding, bonding or otherwise attaching a further layer to the external surface of the highest numbered layer of said air-permeable, non-woven structure incorporating said dissipated absorbent particles.

9. A method as claimed in claim 8, wherein said further layer is:
(a) a layer of non-woven fabric; or
(b) a polymer film.

10. A method as claimed in any of the preceding claims wherein the absorbent particles:
(a) are organic; or
(b) comprise sodium polyacrylate or a polymer blend incorporating sodium polyacrylate; and/or
(c) are hydrophilic

11. A method as claimed in any of the preceding claims, wherein the air-permeable, non-woven structure is compostable in accordance with EN 13432 and or ASTM D6400.

12. A method as claimed in any of the preceding claims wherein the resulting air-permeable, non-woven structure and absorbent particle matrix is further consolidated by the application of heat and/or pressure.

13. A method as claimed in any of the preceding claims wherein the resulting air-permeable, non-woven structure and absorbent particle matrix is subjected to a heated through air process to consolidate the fibres by partial melting and simultaneously to attach said partially melted fibres to said incorporated absorbent particles, thereby to prevent diffusion of the particles from the air-permeable structure.

14. A method as claimed in any of the preceding claims wherein, in step (ii) the dispersion of the absorbent particles is made across:
(a) the entire surface of said air-permeable, non-woven structure; or
(b) selected specific areas of the surface of said air- permeable, non-woven structure.

15. A method as claimed in claim 14 wherein dispersion of the absorbent particles is made across selected specific areas of the surface of said air- permeable, non-woven structure,
further comprising the step, after at least the dispersion (ii) and dissipation (iii) steps, of cutting or otherwise extracting said selected specific areas from the surrounding air permeable substrate;
optionally further comprising the subsequent step of welding or sealing the edges of said extracted selected specific areas on a line on or within 30 millimetres of the cut line.

## Patentansprüche

1. Verfahren zum Auflösen und Einschließen von absorbierenden Partikeln innerhalb luftdurchlässiger Strukturen zur Verwendung bei der Herstellung von absorbierenden Artikeln, wobei das Verfahren die folgenden Schritte umfasst:
(i) Herstellen einer luftdurchlässigen Vliesstruktur, umfassend zumindest eine erste, zweite und dritte Schicht von Vliesstoff, wobei jede der nummerierten Schichten (n) mit jeder darauffolgend nummerierten Schicht (n+1) verklebt, auf dieser hergestellt oder anderweitig mit dieser verbunden ist, und wobei Fasern in jeder der Schichten so angeordnet sind, um Leerräume dazwischen mit vorbestimmter Größe zu definieren, die einem gegebenen Größenverteilungsbereich der absorbierenden Partikel entsprechen;
und wobei die Leerräume in jeder der nummerierten Schicht (n) kleiner sind als die Leerräume in jeder darauffolgend nummerierten Schicht (n + 1);
(ii) Dispergieren von absorbierenden Partikeln durch ein steuerbares mechanisches Mittel auf eine externe Oberfläche der am höchsten nummerierten Schicht der in Schritt (i) ausgebildeten luftdurchlässigen Vliesstruktur, wobei die absorbierenden Partikel einen vorbestimmten Partikelgrößenverteilungsbereich aufweisen;
(iii) Zerstreuen der dispergierten absorbierenden Partikel innerhalb der luftdurchlässigen Vliesstruktur durch Anwenden einer externen Energiequelle, die auf die absorbierenden Partikel in eine Richtung einwirkt, die im Wesentlichen normal zur Ebene der externen Oberfläche der luftdurchlässigen Vliesstruktur steht,
wobei die externe Energiequelle eine Ultraschallschwingungsquelle ist.

2. Verfahren nach Anspruch 1, wobei die Ultraschallschwingungsquelle in Schritt (iii) eine Frequenz im Bereich von zwischen 10 kHz und 50 kHz und eine Amplitude im Bereich von zwischen 5 µm und 500 µm erzeugt.

3. Verfahren nach einem der vorangegangenen Ansprüche, wobei:
(a) die am höchsten nummerierte Schicht der luftdurchlässigen Vliesstruktur Zweikomponentenfasern umfasst, wobei jede Komponente unterschiedliche Wärmeausdehnungseigenschaften aufweist;
(b) jede Schicht der luftdurchlässigen Vliesstruktur, ausgenommen der ersten Schicht, Zweikomponentenfasern umfasst, wobei jede Komponente unterschiedliche Wärmeausdehnungseigenschaften aufweist; und/oder
(c) zumindest eine der Schichten der luftdurchlässigen Vliesstruktur eine Mischung aus zumindest zwei verschiedenen Fasertypen mit unterschiedlichen Wärmeausdehnungseigenschaften umfasst.

4. Verfahren nach Anspruch 3, das weiters nach dem Auflösungsschritt (iii) folgenden Schritt umfasst:
(iv) Aussetzen von zumindest einer Schicht der luftdurchlässigen Vliesstruktur einer externen Wärmequelle und danach Durchführen oder Zulassen des Abkühlens der zumindest einen Schicht, um die absorbierenden Partikel innerhalb der luftdurchlässigen Vliesstruktur einzuschließen.

5. Verfahren nach Anspruch 4, wobei die am höchsten nummerierte Schicht der luftdurchlässigen Vliesstruktur Zweikomponentenfasern umfasst, wobei jede Komponente verschiedene Wärmeausdehnungseigenschaften aufweist,
wobei in Schritt (iv) die am höchsten nummerierte Schicht der luftdurchlässigen Vliesstruktur einer externen Wärmequelle ausgesetzt wird, sodass sich die Komponenten in den Zweikomponentenfasern mit unterschiedlichen Raten beim Erhitzen ausdehnen und beim Kühlen zusammenziehen, wodurch bewirkt wird, dass sich die Fasern kräuseln oder wellen, wodurch die absorbierenden Partikel innerhalb der am höchsten nummerierten Schicht eingeschlossen werden.

6. Verfahren nach Anspruch 4, wobei jede Schicht der luftdurchlässigen Vliesstruktur, ausgenommen der ersten Schicht, Zweikomponentenfasern umfasst, wobei jede Komponente unterschiedliche Wärmeausdehnungseigenschaften aufweist,
wobei in Schritt (iv) alle Schichten der luftdurchlässigen Vliesstruktur einer externen Wärmequelle ausgesetzt werden, sodass sich die Komponenten in den Zweikomponentenfasern mit unterschiedlichen Raten beim Erhitzen ausdehnen und beim Kühlen zusammenziehen, wodurch bewirkt wird, dass sich die Faser kräuseln oder wellen, wodurch die absorbierenden Partikel innerhalb der luftdurchlässigen Vliesstruktur eingeschlossen werden.

7. Verfahren nach Anspruch 4, wobei zumindest eine der Schichten der luftdurchlässigen Vliesstruktur eine Mischung aus zumindest zwei verschiedenen Fasertypen mit unterschiedlichen Wärmeausdehnungseigenschaften umfasst,
wobei in Schritt (iv) alle Schichten der luftdurchlässigen Vliesstruktur einer externen Wärmequelle ausgesetzt werden, sodass sich die Fasern mit der niedrigsten Schmelztemperatur erweichen und klebrig werden, um an benachbarten absorbierenden Partikeln anzuhaften, wodurch die absorbierenden Partikel innerhalb der luftdurchlässigen Vliesstruktur eingeschlossen werden.

8. Verfahren nach einem der vorangegangenen Ansprüche, das ferner den folgenden Schritt umfasst:
(v) Schweißen, Verkleben oder anderweitiges Anbringen einer weiteren Schicht an der externen Oberfläche der am höchsten nummerierten Schicht der luftdurchlässigen Vliesstruktur, die die aufgelösten absorbierenden Partikel einschließt.

9. Verfahren nach Anspruch 8, wobei die weitere Schicht Folgendes ist:
(a) eine Schicht aus einem Vliesstoff; oder
(b) eine Polymerfolie.

10. Verfahren nach einem der vorangegangenen Ansprüche, wobei die absorbierenden Partikel:
(a) organisch sind; oder
(b) Natriumpolyacrylat oder eine Polymermischung, die Natriumpolyacrylat einschließt, umfassen; und/oder
(c) hydrophil sind.

11. Verfahren nach einem der vorangegangenen Ansprüche, wobei die luftdurchlässige Vliesstruktur gemäß EN 13432 und/oder ASTM D6400 kompostierbar ist.

12. Verfahren nach einem der vorangegangenen Ansprüche, wobei die resultierende luftdurchlässige Vliesstruktur und die Matrix aus absorbierenden Partikeln weiters durch die Anwendung von Wärme und/oder Druck vereinigt werden.

13. Verfahren nach einem der vorangegangenen Ansprüche, wobei die resultierende luftdurchlässige Vliesstruktur und die Matrix aus absorbierenden Partikeln einem Lufterhitzungsverfahren unterzogen wird, um die Fasern durch Teilschmelzen zu vereinigen und gleichzeitig die teilgeschmolzenen Fasern an den eingeschlossenen absorbierenden Partikeln anzubringen, wodurch eine Diffusion der Partikeln aus der luftdurchlässigen Struktur verhindert wird.

14. Verfahren nach einem der vorangegangenen Ansprüche, wobei in Schritt (ii) das Dispergieren der absorbierenden Partikeln über Folgendes erfolgt:
(a) über die gesamte Oberfläche der luftdurchlässigen Vliesstruktur; oder
(b) ausgewählte spezifische Bereiche der Oberfläche der luftdurchlässigen Vliesstruktur.

15. Verfahren nach Anspruch 14, wobei das Dispergieren der absorbierenden Partikel über ausgewählte spezifische Bereiche der Oberfläche der luftdurchlässigen Vliesstruktur erfolgt,
ferner umfassend den Schritt des Schneidens oder des anderweitigen Extrahierens der ausgewählten spezifischen Bereiche aus dem umgebenden luftdurchlässigen Substrat nach den Dispergierungs- (ii) und Zerstreungs- (iii) Schritten;
gegebenenfalls ferner umfassend den anschließenden Schritt des Schweißens oder Versiegelns der Ränder der extrahierten spezifischen Bereiche auf einer Linie auf oder innerhalb von 30 mm der Schnittlinie.

## Revendications

1. Procédé de dissipation et de piégeage de particules absorbantes à l'intérieur de structures perméables à l'air, pour utilisation dans la construction d'articles absorbants, ledit procédé comprenant les étapes consistant à :
(i) construire une structure non tissée perméable à l'air comprenant au moins des première, deuxième et troisième couches de tissu non tissé, chaque dite couche numérotée (n) étant liée, fabriquée sur ou d'une autre manière jointe à chaque couche numérotée ultérieurement (n+1), et dans lequel les fibres de chaque dite couche sont agencées de manière à définir entre elles des espaces vides d'une taille prédéterminée, correspondant à une plage de distribution de taille de particule absorbante donnée ;
et dans lequel les espaces vides dans chaque dite couche numérotée (n) sont d'une taille plus petite que les espaces vides dans chaque couche numérotée consécutivement (n+1) ;
(ii) disperser, par l'intermédiaire de moyens mécaniques commandables, des particules absorbantes sur une surface externe de la couche à numéro le plus élevé de ladite structure non tissée perméable à l'air formée à l'étape (i), lesdites particules absorbantes ayant une plage de distribution de taille de particule prédéterminée ;
(iii) dissiper lesdites particules absorbantes dispersées à l'intérieur de la structure non tissée perméable à l'air en appliquant une source d'énergie externe agissant sur les particules absorbantes dans une direction sensiblement normale au plan de la surface externe de la structure non tissée perméable à l'air,
dans lequel la source d'énergie externe est une source de vibrations ultrasonores.

2. Procédé selon la revendication 1, dans lequel la source de vibrations ultrasonores à l'étape (iii) génère une fréquence dans la plage allant de 10 kHz à 50 kHz, et une amplitude dans la plage allant de 5 microns (µm) à 500 microns (µm) .

3. Procédé selon l'une quelconque revendications précédentes, dans lequel :
(a) la couche à numéro le plus élevé de la structure non tissée perméable à l'air comprend des fibres à deux composants, chacun desdits composants présentant des propriétés de dilatation thermique différentes ;
(b) chaque couche de la structure non tissée perméable à l'air, à l'exception de ladite première couche, comprend des fibres à deux composants, chacun desdits composants présentant des propriétés de dilatation thermique différentes ; et/ou
(c) au moins une des couches de la structure non tissée perméable à l'air comprend un mélange d'au moins deux types de fibres différents présentant des propriétés de dilatation thermique différentes.

4. Procédé selon la revendication 3, comprenant en outre l'étape, après l'étape de dissipation (iii), consistant à :
(iv) soumettre au moins une couche de la structure non tissée perméable à l'air à une source de chaleur externe, et ensuite effectuer ou permettre le refroidissement de ladite au moins une couche, de manière à piéger lesdites particules absorbantes à l'intérieur de ladite structure non tissée perméable à l'air.

5. Procédé selon la revendication 4, dans lequel la couche à numéro le plus élevé de la structure non tissée perméable à l'air comprend des fibres à deux composants, chacun desdits composants présentant des propriétés de dilatation thermique différentes,
dans lequel à l'étape (iv), la couche à numéro le plus élevé de la structure non tissée perméable à l'air est soumise à une source de chaleur externe, de telle sorte que lesdits composants dans lesdites fibres à deux composants se dilatent lors d'un chauffage et se contractent lors d'un refroidissement à des vitesses différentielles, en amenant lesdites fibres à s'enrouler ou à se friser, en piégeant ainsi lesdites particules absorbantes à l'intérieur de ladite couche à numéro le plus élevé.

6. Procédé selon la revendication 4, dans lequel chaque couche de la structure non tissée perméable à l'air, à l'exception de ladite première couche, comprend des fibres à deux composants, chaque dit composant présentant des propriétés de dilatation thermique différentes,
dans lequel à l'étape (iv), toutes les couches de la structure non tissée perméable à l'air sont soumises à une source de chaleur externe, de telle sorte que lesdits composants dans lesdites fibres à deux composants se dilatent lors d'un chauffage et se contractent lors d'un refroidissement à des vitesses différentielles, en amenant les fibres à s'enrouler ou à se friser, en piégeant ainsi lesdites particules absorbantes à l'intérieur de ladite structure non tissée perméable à l'air.

7. Procédé selon la revendication 4, dans lequel au moins une des couches de la structure non tissée perméable à l'air comprend un mélange d'au moins deux types de fibre différents présentant des propriétés de dilatation thermique différentes,
dans lequel à l'étape (iv), toutes les couches de la structure non tissée perméable à l'air sont soumises à une source de chaleur externe, de sorte que les fibres présentant la température de fusion la plus basse se ramollissent et deviennent collantes de manière à adhérer à des particules absorbantes adjacentes, en piégeant ainsi lesdites particules absorbantes à l'intérieur de ladite structure non tissée perméable à l'air.

8. Procédé selon l'une quelconque des revendications précédentes, comprenant en outre l'étape consistant à :
(v) souder, lier ou fixer d'une autre manière une couche supplémentaire à la surface externe de la couche à numéro le plus élevé de ladite structure non tissée perméable à l'air incorporant lesdites particules absorbantes dissipées.

9. Procédé selon la revendication 8, dans lequel ladite couche supplémentaire est :
(a) une couche de tissu non tissé ; ou
(b) un film polymère.

10. Procédé selon l'une quelconque des revendications précédentes, dans lequel les particules absorbantes :
(a) sont organiques ; ou
(b) comprennent du polyacrylate de sodium ou un mélange de polymères incorporant du polyacrylate de sodium ; et/ou
(c) sont hydrophiles.

11. Procédé selon l'une quelconque des revendications précédentes, dans lequel la structure non tissée perméable à l'air est compostable conformément à la norme EN 13432 et/ou à la norme ASTM D6400.

12. Procédé selon l'une quelconque des revendications précédentes, dans lequel la structure non tissée perméable à l'air et la matrice de particules absorbantes résultantes sont en outre consolidées par l'application de chaleur et/ou de pression.

13. Procédé selon l'une quelconque des revendications précédentes, dans lequel la structure non tissée perméable à l'air et la matrice de particules absorbantes résultantes sont soumises à un processus de chauffage à travers l'air pour consolider les fibres par fusion partielle et simultanément pour fixer lesdites fibres partiellement fondues auxdites particules absorbantes incorporées, afin d'empêcher ainsi la diffusion des particules à partir de la structure perméable à l'air.

14. Procédé selon l'une quelconque des revendications précédentes, dans lequel, dans l'étape (ii), la dispersion des particules absorbantes est effectuée à travers :
(a) toute la surface de ladite structure non tissée perméable à l'air ; ou
(b) des zones spécifiques sélectionnées de la surface de ladite structure non tissée perméable à l'air.

15. Procédé selon la revendication 14, dans lequel la dispersion des particules absorbantes est effectuée sur des zones spécifiques sélectionnées de la surface de ladite structure non tissée perméable à l'air,
comprenant en outre l'étape, après au moins les étapes de dispersion (ii) et de dissipation (iii), de couper ou d'extraire de toute autre manière lesdites zones spécifiques sélectionnées à partir du substrat perméable à l'air environnant ;
comprenant en outre facultativement l'étape ultérieure consistant à souder ou à sceller les bords desdites zones spécifiques sélectionnées extraites sur une ligne sur ou à moins de 30 millimètres de la ligne de découpe.
